(19) Europäisches Patentamt / European Patent Office / Office européen des brevets

(11) **EP 3 513 154 B1**

(12) **EUROPÄISCHE PATENTSCHRIFT**

(45) Veröffentlichungstag und Bekanntmachung des Hinweises auf die Patenterteilung:
**17.03.2021 Patentblatt 2021/11**

(21) Anmeldenummer: **17772320.2**

(22) Anmeldetag: **06.09.2017**

(51) Int Cl.:
*G01H 3/04* (2006.01)     *G01H 15/00* (2006.01)
*G01N 29/00* (2006.01)     *G01N 29/024* (2006.01)
*G01N 29/032* (2006.01)     *G01N 29/34* (2006.01)
*G01N 29/44* (2006.01)     *G01N 33/28* (2006.01)

(86) Internationale Anmeldenummer:
**PCT/EP2017/072287**

(87) Internationale Veröffentlichungsnummer:
**WO 2018/050500 (22.03.2018 Gazette 2018/12)**

(54) **VERFAHREN UND VORRICHTUNG ZUR BESTIMMUNG UND/ODER ÜBERWACHUNG DER DURCHSCHLAGSPANNUNG EINES TRANSFORMATORENÖLS**

METHOD AND DEVICE FOR DETERMINING AND/OR MONITORING THE BREAKDOWN VOLTAGE OF A TRANSFORMER OIL

PROCÉDÉ ET DISPOSITIF POUR DÉTERMINER ET/OU CONTRÔLER LA TENSION DE CLAQUAGE D'UNE HUILE DE TRANSFORMATEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.09.2016 DE 102016117188**

(43) Veröffentlichungstag der Anmeldung:
**24.07.2019 Patentblatt 2019/30**

(73) Patentinhaber: **Passerro GmbH**
**04299 Leipzig (DE)**

(72) Erfinder: **WROBEL, Matthias**
**81243 München (DE)**

(74) Vertreter: **advotec.**
**Patent- und Rechtsanwälte**
**Beethovenstrasse 5**
**97080 Würzburg (DE)**

(56) Entgegenhaltungen:
**DE-A1- 19 706 486     DE-A1-102013 005 003**
**DE-A1-102014 104 963**

# Beschreibung

Die Erfindung betrifft ein Verfahren und eine Vorrichtung zur Bestimmung und/oder Überwachung der Durchschlagsspannung eines Transformatorenöls nach dem Obergriff der unabhängigen Ansprüche.

Vorrichtungen in der Hochspannungstechnik, wie beispielsweise Transformatoren, Kondensatoren, Petersenspulen und/oder Schalter, sind bereits aus dem Stand der Technik bekannt und dienen insbesondere, jedoch keineswegs ausschließlich, der Gewährleistung einer kontinuierlichen elektrischen Energieversorgung. Unter diesen Vorrichtungen zählen die Transformatoren zu den wichtigsten und auch kostenintensivsten Betriebsmitteln in der elektrischen Energieversorgung. Um die kontinuierliche und fehlerfreie Versorgung mit elektrischer Energie sicherzustellen sowie auch wirtschaftliche Schäden zu vermeiden, ist es wichtig, eventuell auftretende Fehler im Betrieb eines Transformators, welche Ausfälle verursachen können, rechtzeitig zu erkennen, um geeignete Maßnahmen zur Fehlerbeseitigung einzuleiten.

Typischerweise wird in Transformatoren eine Kombination aus einem flüssigen und einem festen Isolierstoff als Isoliermittel verwendet. Das feste Isoliermittel ist beispielsweise, jedoch keineswegs ausschließlich, Zellulosepapier und/oder eine Spanplatte. Das flüssige Isoliermittel, das Transformatorenöl, ist auch bei hohen Temperaturen stabil und dient der Isolation, der Funkenlöschung, der Schmierung und/oder der Kühlung des Transformators. Fehler an dem festen und/oder flüssigen Isolierstoff resultieren, insbesondere an derartigen flüssigkeitsgefüllten Transformatoren, dabei fast ausschließlich aus der Entwicklung von in dem Transformatorenöl gelösten Gasen sowie einer dadurch bedingten Erhöhung des Wassergehaltes. Ein Grund für die Entstehung der Gase ist beispielsweise die Zersetzung fester und/oder flüssiger Isolierstoffe, welche durch Teilentladung und Kreisströme, örtliche Überhitzungen durch Kurzschluss, hohe Übergangswiderstände, starke Wirbelströme sowie durch Lichtbogenentladungen und/oder Lichtbogenüberschläge hervorgerufen werden können. Durch die elektrische und/oder thermische Energiezufuhr erfolgt eine Zerstörung der langkettigen Ölmoleküle, wodurch insbesondere Wasserstoff und leichte Kohlenwasserstoffverbindungen entstehen. Zusätzlich entstehen bei der Zersetzung von Zellulose Kohlenmonoxid und Kohlendioxid, welche je nach Menge der entstandenen Gase in gelöster und/oder ungelöster Form auftreten können. So ist es auch möglich, dass Wassermoleküle entstehen, welche zu der unerwünschten Ölfeuchte führen.

Ein weiteres Problem des in dem Transformatorenöl enthaltenen Wassers besteht darin, dass das Wasser in das feste Isoliermittel, wie beispielsweise Zellulosepapier und/oder die Spanplatte, eindringt und die produktionsbedingt darin enthaltenen Säuren in das Transformatorenöl auswäscht. Dadurch kommt es zu einer zusätzlichen Belastung des Transformators, wobei diese Belastung, bedingt durch mannigfaltige Ursachen, wie beispielsweise Tagesablauf-bedingte Temperaturschwankungen (z.B. zwischen Tag und Nacht), wechselnd stark oder schwach ist.

Daher ist es zur Erhaltung der Funktionalität und/oder der Gewährleistung einer kontinuierlichen elektrischen Energieversorgung wichtig, die Durchschlagspannung des Transformatorenöls zu bestimmen und/oder zu überwachen. Es ist daher nicht verwunderlich, dass aus dem Stand der Technik eine Vielzahl verschiedener Verfahren und Vorrichtungen zur Bestimmung der Durchschlagspannung von Transformatorenölen bekannt ist, welche beispielsweise die in dem Transformatorenöl gelösten Gase und/oder die Wasserkonzentration bestimmen, da beide bekanntermaßen einen wesentlichen Einfluss auf die Durchschlagspannung und somit indirekt auf die Lebensdauer und/oder Nutzungszeit des Transformators haben. Dies liegt darin begründet, dass Wasser im Transformator zur Hydrolyse des festen Isoliermittels und somit zur Erniedrigung dessen Polymerisationsgrades führt. Allerdings weisen diese Verfahren und Vorrichtungen allesamt den Nachteil auf, dass eine Probenentnahme notwendig ist und die Bestimmung und/oder Überwachung der Durchschlagspannung des Transformatorenöls weder permanent noch online erfolgt. Auf diese Weise ist es auch nicht möglich, die Belastungsspitzen des Transformators zu erkennen. Ein weiteres Problem hierbei liegt darin, dass Transformatorenöl sehr stark hygroskopisch ist, so dass bereits die Probenentnahme zu einer Verfälschung der Messwerte führt.

Die deutsche Offenlegungsschrift DE 197 06 486 A1 betrifft eine Vorrichtung und ein Verfahren zum Bestimmen des Alterungszustands eines flüssigen Mediums, insbesondere eines Öls, ausgehend von einem Ausgangszustand desselben. Dabei wird zumindest ein Zustandsparameter des flüssigen Mediums während einer ersten Periode, in der das flüssige Medium den Ausgangszustand aufweist, während zumindest einer zweiten, zeitlich nachfolgenden Periode erfasst. Nachfolgend werden die während der ersten und der zweiten Periode erfassten Zustandsparameter verglichen.

Die deutsche Offenlegungsschrift DE 10 2014 104 963 A1 betrifft ein Verfahren zur quasi kontinuierlichen Überwachung und Messung des Zustands ölgefüllter Transformatoren und/oder Geräten, zur Bewertung deren Isolation, zur Überwachung von Isolierölen und zur Überwachung elektrischer Betriebsmittel, in denen Isolieröle eingesetzt werden. Dabei wird quasi kontinuierlich die Durchschlagsspannung, die Feuchte, die Versauerung, die Neutralisationszahl und/oder der Verlustfaktor des Isolieröls bestimmt, sowie die elektrische Leitfähigkeit von eingesetzten Isolierölen, die Dielektrizitätszahl und die Isolieröltemperatur gemessen. Aus den gemessenen Größen kann schließlich die elektrische Durchschlagfestigkeit, der Verlustfaktor, die Feuchte des Isolieröls, dessen Neutralisationszahl und Versauerung ermittelt werden.

Die deutsche Offenlegungsschrift DE 10 2013 005 003 A1 betrifft ein Verfahren zur Begrenzung von Verschleiß in Industriegetrieben, Windenergiegetrieben, Turbinen, Hydraulikanlagen, Transformatoren, Ölalterung und/oder die Bestimmung der Alterung beziehungsweise Veränderung von Kühlschmierstoffen oder Treibstoffen und/oder Isolierölen. Dabei wird über den Gradienten der, über eine selbstlernende, adaptive Temperaturkompensation mit zeitlich abklingender Gewichtung früherer Messwerte, temperaturkompensierten elektrischen Leitfähigkeit der momentane Getriebeverschleiß gemessen.

Es besteht daher ein großer Bedarf an einem Verfahren und einer Vorrichtung zur Bestimmung und/oder Überwachung der Durchschlagspannung eines Transformatorenöls, und damit indirekt zur Bestimmung und/oder Überwachung der Durchschlagspannung einer Vorrichtung in der Hochspannungstechnik, mittels welchem bzw. welcher eine schnelle, zuverlässige und hinreichend genaue Bestimmung und/oder Überwachung der Durchschlagspannung gewährleistet ist, um einen unnötigen und auch kostenintensiven Ölwechsel zu vermeiden und gleichzeitig die kontinuierliche elektrische Energieversorgung sicherzustellen. Zudem sollten das Verfahren und die Vorrichtung kostengünstig realisierbar, zuverlässig arbeitend und für eine dauerhafte Bestimmung und/oder Überwachung geeignet sein. Die Erfindung hat sich daher die Aufgabe gestellt, ein Verfahren und eine Vorrichtung zur Bestimmung und/oder Überwachung der Durchschlagspannung eines Transformatorenöls bereitzustellen, um die oben genannten Schwierigkeiten zu überwinden und um vor allem einen vorzeitigen und/oder unnötigen Ölwechsel zu vermeiden sowie eine Ölregeneration und/oder die wartungs- und/oder reparaturbedingten Arbeiten optimal zu planen, um dadurch die Stillstandzeiten der Vorrichtung und die dadurch entstehenden Kosten auf ein Minimum zu reduzieren.

Diese Aufgabe wird auf überraschend einfache aber wirkungsvolle Weise durch ein Verfahren zur Bestimmung und/oder Überwachung der Durchschlagspannung eines Transformatorenöls sowie eine entsprechende Vorrichtung nach der Lehre der unabhängigen Hauptansprüche gelöst.

Erfindungsgemäß ist ein Verfahren zur Bestimmung und/oder Überwachung der Durchschlagspannung eines Transformatorenöls vorgeschlagen, welches die folgenden Schritte umfasst:

a) Durchführen einer akustischen Impedanzmessung des Transformatorenöls, wobei die Impedanz eines teilweise oder vollständig in dem Transformatorenöl angeordneten, zur Eigen-Schwingung und/oder Schwingungsübertragung auf das Transformatorenöl geeigneten Mittels in mindestens einem Frequenzband definierter Frequenzbreite bestimmt wird; und

b) Berechnen eines Resonatorqualitätsfaktors für das Frequenzband basierend auf der in Schritt a) durchgeführten Bestimmung; und

c) Durchführen einer Kalibrierung und Berechnen eines akustischen Ungleichgewichtes des Transformatorenöls basierend auf den in der Kalibrierung ermittelten Kalibrierwerten und auf der in Schritt b) durchgeführten Berechnung; und

d) Ermitteln der Durchschlagspannung des Transformatorenöls basierend auf der in Schritt c) durchgeführten Berechnung.

Das erfindungsgemäße Verfahren beruht auf dem Grundgedanken, dass die Kombination mehrerer physikalischer Zusammenhänge für eine online durchführbare, hinreichend genaue Bestimmung und/oder Überwachung der Durchschlagspannung eines Transformatorenöls angewendet werden kann. So ist einerseits erkannt worden, dass die Durchschlagspannung eines Transformatorenöls eine Funktion in Abhängigkeit der Wasserkonzentration und der Gesamtsäurezahl des Transformatorenöls ist. Weiterhin ist erkannt worden, dass das akustische Ungleichgewicht eines Transformatorenöls eine Funktion in Abhängigkeit der Viskosität des Transformatorenöls ist, wobei die Viskosität und die Oberflächenspannung in einem Öl voneinander abhängige Größen sind. Überdies ist erkannt worden, dass die Oberflächenspannung eine Funktion des Verhältnisses in Abhängigkeit der Wasserkonzentration und der Gesamtsäurezahl des Transformatorenöls ist. Erfindungsgemäß ist dabei erkannt worden, dass die Durchschlagspannung eines Transformatorenöls eine Funktion in Abhängigkeit des akustischen Ungleichgewichtes des Transformatorenöls ist, wobei eine oder weitere Abhängigkeiten verschiedener physikalischer Größen bei der Durchführung des erfindungsgemäßen Verfahrens vernachlässigbar sind und/oder zu einem tolerierbaren Fehler führen.

Somit ist im Rahmen der vorliegenden Erfindung erkannt worden, dass zur hinreichend genauen Bestimmung der Durchschlagspannung eines Transformatorenöls die Kombination aus der Durchführung einer akustischen Impedanzmessung des Transformatorenöls, der Berechnung eines Resonatorqualitätsfaktors und der Berechnung eines akustischen Ungleichgewichtes des Transformatorenöls ausreichend ist.

Der Begriff "Verfahren zur Bestimmung und/oder Überwachung der Durchschlagspannung" betrifft ein Verfahren zur einmaligen oder wiederholten Ermittlung der Durchschlagspannung des Transformatorenöls. Bevorzugt basiert das Verfahren auf der Ermittlung der Veränderung, bevorzugt eine Verbesserung oder eine Verschlechterung der Durchschlagspannung des Transformatorenöls. Noch mehr bevorzugt wird diese Veränderung über die Zeit ermittelt, bevorzugt die Nutzungszeit, die Standzeit und/oder die Stillstandzeit. Weiterhin bevorzugt wird die Durchschlagspannung des Transformatorenöls in einem regelmäßigen oder einem unregelmäßigen Intervall oder dauerhaft bestimmt, um die Veränderung der Durchschlagspannung sehr schnell erfassen zu können. Dies ist insbesondere wichtig, da es sich bei Transformatorenöl um kein statisches System handelt. Zudem lässt sich verfolgen, unter welchen Bedingungen und/oder Einflüssen die Veränderung der Durchschlagspannung des Transformatorenöls fortschreitet bzw. verlangsamt ist. Überdies lässt sich die Entstehung und/oder Ursache dieser Veränderung aufzeigen, so dass ein bevorstehendes Wartungsintervall und/oder ein bevorstehender Transformatorenöl-Wechsel bzw. eine entsprechende Transformatorenöl-Regeneration bestmöglich geplant und/oder vorhergesagt werden kann. Dabei ist es möglich, dass das erfindungsgemäße Verfahren zusätzliche Schritte enthalten kann, welche nach oder zwischen den explizit aufgeführten essentiellen Schritten a) bis d) liegen. Bevorzugt ist das Verfahren automatisierbar.

Der Begriff "Bestimmung der Durchschlagspannung" des Transformatorenöls betrifft die Ermittlung der aktuellen Durchschlagspannung des Transformatorenöls. Die Bestimmung erfolgt bevorzugt semiquantitativ, quantitativ, direkt und/oder indirekt. So ist es beispielsweise möglich, mittels der Ermittlung der Durchschlagspannung des Transformatorenöls indirekt den Zustand des Transformators zu ermitteln.

Der Begriff "Überwachung der Durchschlagspannung" betrifft die Verfolgung und/oder die Vorhersage der ermittelten Durchschlagspannung des Transformatorenöls. Die Überwachung ist beispielsweise, jedoch keineswegs ausschließlich, nummerisch und/oder graphisch darstellbar. Zur Erhöhung der Genauigkeit der Überwachung erfolgt diese dabei bevorzugt in einem regelmäßigen oder einem unregelmäßigen Intervall oder dauerhaft. Der Vorteil einer länger dauernden Überwachung liegt darin, dass eine Vorhersage der Durchschlagspannung des Transformatorenöls drastisch verbessert ist.

Es ist einem Fachmann verständlich, dass eine Bestimmung und/oder eine Überwachung in der Regel nicht zu 100 Prozent korrekt sein kann. Der Begriff betrifft daher eine statistisch signifikante Wahrscheinlichkeit, was die Genauigkeit der Ermittlung der Durchschlagspannung bzw. der Verfolgung und/oder Vorhersage der ermittelten Durchschlagspannung betrifft. Ob eine derartige Bestimmung und/oder Überwachung statistisch signifikant ist, kann, ohne erfinderisch tätig zu werden, von einem Fachmann mittels in der Fachwelt bekannter Verfahren bestimmt werden. Beispielsweise sind statistische Evaluierungstools zu nennen, wie beispielsweise die Beurteilung des Konfidenzintervalls, des P-Wertes, des Student's T-Tests, der Mann-Whitney-Bestimmung usw. Die entsprechenden Intervalle sind mindestens 90 %, mindestens 95 %, mindestens 97 %, mindestens 98 % oder mindestens 99 % korrekt. Die P-Werte sind bevorzugt 0,1, 0,05, 0,01, 0,005 oder 0,0001. Bevorzugt ist die Bestimmung und/oder die Überwachung der Durchschlagspannung im Rahmen der vorliegenden Erfindung zu mindestens 80 %, mindestens 90 %, mindestens 95 %, mindestens 96 %, mindestens 97 %, mindestens 98 %, mindestens 99 %, mindestens 99,5 % oder 100 % korrekt.

Der Begriff "Durchschlagspannung", abgekürzt BDV (engl. *breakdown voltage*) betrifft diejenige elektrische Feldstärke im Transformatorenöl, welche in diesem höchstens herrschen darf, ohne dass es zu einem Spannungsdurchschlag, Lichtbogen und/oder Funkenschlag und den damit verbundenen Ausfällen sowie Nachteilen kommt. Die Durchschlagspannung ist von verschiedenen Faktoren abhängig. Die Durchschlagspannung ist bevorzugt graphisch und/oder in Form eines absoluten Wertes darstellbar, welcher konform mit der gültigen DIN-Norm ist, wie beispielsweise mit der DIN EN 60243-1:2012-05.

Das erfindungsgemäße Verfahren umfasst einen Schritt a) zum Durchführen einer akustischen Impedanzmessung des Transformatorenöls, wobei die Impedanz eines teilweise oder vollständig in dem Transformatorenöl angeordneten, zur Eigen-Schwingung und/oder Schwingungsübertragung auf das Transformatorenöl geeigneten Mittels in mindestens einem Frequenzband definierter Frequenzbreite bestimmt wird.

Der Begriff "akustische Impedanzmessung" betrifft die akustische Untersuchung eines Mediums durch Rückschlüsse aus den Veränderungen des Resonanzverhaltens eines teilweise oder vollständig in dem Medium angeordneten, zur Eigen-Schwingung und/oder Schwingungsübertragung auf das Medium geeigneten Mittels, wie beispielsweise ein Resonator, ein Resonanzkörper, eine Resonanzkammer oder ein Wandler, im Ultraschallfrequenzbereich (20 kHz - 1 GHz). Die Veränderungen basieren dabei auf den Wechselwirkungen der in dem Medium enthaltenen Moleküle mit den elastischen Wellen und/oder den Schwingungen des genannten Mittels. Dabei ist es einem Fachmann verständlich, dass das Zusammenspiel aller Komponenten kalibriert wird. Auf diese Weise ist es möglich, mittels der akustischen Impedanzmessung die Zusammensetzung des Mediums zu untersuchen bzw. auf die Zusammensetzung des Mediums zu schließen. Das zu untersuchende Medium ist im Rahmen der Erfindung bevorzugt ein Transformatorenöl.

Im Rahmen der vorliegenden Erfindung ist erkannt worden, dass, wenn sich die elastische Welle durch das Transformatorenöl ausbreitet und/oder ein entsprechendes Mittel in dem Transformatorenöl angeordnet ist, in welchem ein molekulares Gleichgewicht vorherrscht, das Gleichgewicht von der Wirkung der Frequenz der Welle und/oder Schwingung abhängig ist. Dabei ist es wesentlich, dass die Periode der elastischen Welle und/oder der Schwingung viel länger ist, als die Relaxationszeit für die Gleichgewichtslageveränderung, welche nachfolgend durch die Welle und/oder die Schwingung gestört wird. Ist dagegen die Periode der Welle und/oder der Schwingung viel kürzer als die Relaxationszeit, so wird die Welle und/oder die Schwingung das Gleichgewicht nicht stören. Folglich bleibt das Gleichgewicht ungestört. In dem Fall, dass die Periode mit der Relaxationszeit vergleichbar ist, treten Änderungen in der Ausbreitungsgeschwindigkeit und dem Absorptionskoeffizienten der elastischen Welle und/oder der Schwingung auf. Aus den Messungen der Veränderungen können beispielsweise, jedoch keinesfalls ausschließlich, die Relaxationszeit und die Geschwindigkeitskonstanten des Gleichgewichts bestimmt werden, sowie die Durchlaufzeit, die Frequenzverschiebung und/oder die Dämpfung.

Bevorzugt betrifft die akustische Impedanzmessung Ultraschallfrequenzen, noch mehr bevorzugt Frequenzen von 75 kHz bis 750 kHz. Weiter bevorzugt wird die akustische Impedanzmessung in mindestens einem Frequenzband durchgeführt. Noch mehr bevorzugt wird die akustische Impedanzmessung in zwei, drei, vier, fünf, sechs, sieben, acht, neun, zehn oder mehr Frequenzbändern durchgeführt, wobei im Rahmen der Erfindung als wesentlich erkannt worden ist, dass jedes Frequenzband eine definierte Frequenzbreite, noch mehr bevorzugt in einem definierten Frequenzbereich, aufweist. Es ist daher verständlich, dass jedes Frequenzband dieselbe Frequenzbreite aufweist.

In einem weiteren Schritt wird ein Resonatorqualitätsfaktor für das Frequenzband basierend auf der zuvor durchgeführten Bestimmung berechnet. Der Begriff "Resonatorqualitätsfaktor", abgekürzt Q-Faktor, ist ein dimensionsloser Parameter, welcher den Grad der Unterdämpfung eines zur Eigen-Schwingung und/oder Schwingungsübertragung auf das Transformatorenöl geeigneten Mittels beschreibt und daher folglich die Bandbreite desselben bezogen auf seine Mittenfrequenz kennzeichnet.

Im Rahmen der Erfindung ist zudem erkannt worden, dass es für kleine Dämpfungswerte *a* (hohe Impedanzwerte) möglich ist, die bereits aus dem Stand der Technik bekannte Beziehung zwischen der Halbleistungsbandbreite (HPB) oder der "3dB Bandbreite" Δ*f* von einer bestimmten Amplitude und der Frequenz $f_n$ mit der maximalen Breite zu bestimmen. Diesbezüglich gilt die folgende Formel (1):

$$\frac{\alpha\lambda}{\pi} = \frac{\Delta f}{fn} = \frac{1}{Q}$$

wobei

*a* der Dämpfungswert, und
Δ*f* die Breite der Amplitude, und
$f_n$ die Frequenz mit maximaler Breite, und
Q der Resonatorqualitätsfaktor ist.

Bevorzugt ergibt sich gemäß der Formel (1), dass der Resonatorqualitätsfaktor Q eines idealen Resonators mit Flüssigkeitsdämpfung erhältlich ist. Der Resonatorqualitätsfaktor Q des reellen Resonanzsystems $Q_{real}$ ist dabei umgekehrt proportional zum Gesamtenergieverlust im Resonanzsystem, das bedeutet, der Beitrag aus allen Arten von Energieverlusten, wie Flüssigkeitsdämpfung und zusätzliche Verluste aus Strahldivergenz, Streuung, Reibungseffekte, unvollkommene Reflexion an der Oberfläche und/oder Kopplungsverluste.

Weiter bevorzugt erfolgt die Berechnung des Resonatorqualitätsfaktors mittels der Formel (2):

$$Q_n = \frac{f_0}{\Delta f}$$

wobei

$Qn$ der Resonatorqualitätsfaktor für das Frequenzband, und
$f_0$ die Frequenz mit der maximalen Amplitude, und
Δ*f* die definierte Resonanzbreite in Hz ist.

Des Weiteren wird ein akustisches Ungleichgewicht, abgekürzt AcDis (engl. *acustic disbalance*), des Transformatorenöls basierend auf der zuvor durchgeführten Berechnung des Resonatorqualitätsfaktors berechnet. Bevorzugt erfolgt die Berechnung des akustischen Ungleichgewichtes mittels der Formel (3):

$$AcDis = a + b * Q_n$$

wobei

*AcDis* das akustische Ungleichgewicht, und

*a* eine empirisch ermittelbare Konstante bzw. Kalibrierwert, und

*b* eine empirisch ermittelbare Konstante bzw. Kalibrierwert, und

*Qn* der Resonatorqualitätsfaktor für das Frequenzband ist.

Es ist einem Fachmann verständlich, dass die Kalibrierwerte *a* bzw. *b* von dem zur Eigen-Schwingung und/oder Schwingungsübertragung auf das Transformatorenöl geeigneten Mittel, wie einem Resonator und/oder dem benutzen Piezomaterial, abhängen, so dass diese erst in dem Kalibrierungsprozess ermittelt werden. In welcher Weise die Kalibrierung durchgeführt wird, ist grundsätzlich beliebig und unterliegt dem fachmännischen Können.

Erfindungsgemäß ist erkannt worden, dass die Durchschlagspannung eines Transformatorenöls eine Funktion in Abhängigkeit des akustischen Ungleichgewichtes des Transformatorenöls ist, wobei eine oder weitere Abhängigkeiten verschiedener physikalischer Größen bei der Durchführung des erfindungsgemäßen Verfahrens vernachlässigbar sind und/oder zu einem tolerierbaren Fehler führen. Daher kann die Durchschlagspannung des Transformatorenöls basierend auf der zuvor durchgeführten Berechnung des akustischen Ungleichgewichtes ermittelt werden.

Im Rahmen der Erfindung werden die Bestimmung, die Berechnung und/oder die Ermittlung bevorzugt computerunterstützt durchgeführt. Für eine computerunterstützte Durchführung dieser Schritte, beispielsweise der Schritte a), b), c) und/oder d), sind alle einem Fachmann bekannten Mittel denkbar, wie beispielsweise Computer und/oder ein Computerprogramm. Ein Computerprogramm kann zusätzlich das entsprechende Ergebnis evaluieren, beispielsweise automatisch eine Beurteilung des Wertes liefern. Es ist weiterhin beispielsweise denkbar, dass die Schritte a), b), c) und/oder d) von einer Auswerte-, einer Analyse- und/oder einer Evaluierungseinheit unterstützt sind. Bevorzugt ist es auch möglich, zeitlich aufeinanderfolgende Werte in einem Vergleich zu berücksichtigen, so dass basierend auf diesem Vergleich eine Vorhersage getroffen werden kann, wie sich die Durchschlagspannung in zeitlicher Abhängigkeit verändert. Somit ist es denkbar, dass eine geringe und nicht signifikante, eine große und signifikante und/oder keine Veränderung der zeitlich aufeinander folgenden Werte indikativ für eine bestimmte Durchschlagspannung des Transformators ist. Eine Veränderung der zeitlich aufeinander folgenden Werte kann bevorzugt eine Verbesserung und/oder eine Verschlechterung dieser sein. In diesem Zusammenhang ist es denkbar, dass das Ergebnis des Vergleichs als zeitliche Angabe, beispielsweise in Jahren, Monaten, Tagen, Stunden und/oder Minuten, in einem absoluten Wert und/oder einem relativen Wert ausgebbar ist.

Der Begriff "Vergleich" betrifft, wie er hier angewendet wird, den Vergleich sich entsprechender Parameter und/oder Werte. So ist es beispielsweise denkbar, dass absolute Werte miteinander verglichen werden. Gleiches gilt für relative Werte und/oder ein Intensitätssignal. Es ist auch denkbar, den Vergleich auf der Basis eines empirisch ermittelten Modells für Referenz-Transformatorenöle durchzuführen.

Der Begriff "Transformatorenöl" betrifft einen bei hohen Temperaturen stabilen, flüssigen Isolierstoff, welcher der Isolation, der Funkenlöschung, der Schmierung und/oder der Kühlung einer Vorrichtung in der Hochspanungstechnik, wie beispielsweise eines Transformators, eines Kondensators und/oder eines Schalters, dient. Der flüssige Isolierstoff ist beispielweise, jedoch keineswegs ausschließlich, ein hochraffiniertes Mineralöl, ein verflüssigtes Gas (GTL), ein dünnflüssiges Silikonöl, ein Natural-Öl, ein Pflanzenöl, ein synthetischer organischer Ester, wie ein gesättigter Pentaerythrittetrafettssäureester, und/oder eine Aminosäureverbindung.

Mittels des erfindungsgemäßen Verfahrens ist es somit möglich, die Durchschlagspannung des Transformatorenöls einfach, schnell und zuverlässig zu bestimmen und/oder zu überwachen, um beispielsweise eine Aussage über die Durchschlagspannung der entsprechenden Vorrichtung in der Hochspannungstechnik geben zu können. Dabei ist es möglich, diese Bestimmung und/oder Überwachung bei einer im Betrieb befindlichen Vorrichtung, d.h. online, durchzuführen. Vorteilhafterweise ist das Verfahren derart ausgestaltet, dass auf eine Probenentnahme mit den bekannten Nachteilen vollständig verzichtet werden kann. Auf diese Weise ist es möglich, einen vorzeitigen und/oder unnötigen sowie kostenintensiven Ölwechsel zu vermeiden und gleichzeitig die kontinuierliche elektrische Energieversorgung zu jeder Zeit sicherzustellen. Zudem ist es möglich, die für eine Wartung, Regeneration und/oder Reparatur erforderlichen Stillstandzeiten der Vorrichtung optimal zu planen, um unnötige Stillstandzeiten und/oder Kosten zu vermeiden.

Vorteilhafte Weiterbildungen der Erfindung, welche einzeln oder in Kombination realisierbar sind, sind in den Unteransprüchen dargestellt.

In einer Weiterbildung der Erfindung ist es denkbar, dass nach dem Schritt c) ein zusätzlicher Schritt cl) umfasst ist:

c1) Erfassen mindestens eines Wertes mindestens einer charakteristischen physikalischen Eigenschaft des Transformatorenöls.

Im Rahmen der vorliegenden Erfindung ist des Weiteren erkannt worden, dass die einzelne Information zu dem akustischen Ungleichgewicht des Transformatorenöls zwar zur hinreichend genauen Ermittlung der Durchschlagspannung ausreichend ist, allerdings ist auch erkannt worden, dass zur Fixierung des "Verlagerungspunkts" mindestens ein Wert mindestens einer charakteristischen physikalischen Eigenschaft des Transformatorenöls zu erfassen und bei dieser Ermittlung heranzuziehen ist. Es ist insbesondere erkannt worden, dass in dem Fall, wenn das akustische Ungleichgewicht des Transformatorenöls entlang einer Achse einer 2-dimensionalen Funktion, d.h. beispielsweise einer Kurve aus Gesamtsäuregehalt und Wasserkonzentration, "gleitet", nur der mindestens eine Wert mindestens einer charakteristischen physikalischen Eigenschaft das Verhalten der Funktion zu einer verbesserten hinreichend genauen Ermittlung der Durchschlagspannung erzwingt. Es ist daher verständlich, dass die Durchschlagspannung des Transformatorenöls basierend auf dem berechneten akustischen Ungleichgewicht und dem erfassten mindestens einen Wert der mindestens einen charakteristischen Eigenschaft ermittelt wird, wobei gilt, dass die Genauigkeit der Ermittlung in Schritt d) mit zunehmender Anzahl an erfassten Werten steigt.

Der Begriff "charakteristische physikalische Eigenschaft" betrifft dabei eine für das Transformatorenöl wesentliche physikalische Eigenschaft, mittels welcher ein direkter oder indirekter Rückschluss auf die Durchschlagspannung des Transformatorenöls bzw. den Zustand des Transformators möglich ist. Bevorzugt verändert sich diese Eigenschaft in Abhängigkeit des Alterungsprozesses des Transformatorenöls, wobei die Veränderung bevorzugt eine Verbesserung oder eine Verschlechterung ist. Einem Fachmann sind derartige charakteristische physikalische Eigenschaften des Transformatorenöls sowie deren Erfassung, Bestimmung und/oder Berechnung gut bekannt, wie beispielsweise die Dichte, die Farbe, der Brechungsindex, die Temperatur, die Löslichkeit in Wasser, die Wasserkonzentration, die Oberflächenspannung, die Viskosität, die relative und/oder absolute Feuchte bzw. Sättigung, der Verlustfaktor, der Säuregehalt, die elektrische Konstante, die elektrische Leitfähigkeit und/oder die Konzentration mindestens eines Fluides. Zudem sind weitere, hier nicht aufgeführte, Eigenschaften denkbar, wie ein Resonatorqualitätsfaktor für ein Frequenzband und/oder ein akustisches Ungleichgewicht für ein Transformatorenöl.

Dabei ist es denkbar, dass 2, 3, 4, 5, 6, 7, 8, 9, 10, 11, 12, 13, 14, 15, 16, 17, 18, 19, 20 oder mehr Werte derselben charakteristischen physikalischen Eigenschaft erfasst werden. Bevorzugt ist es denkbar, dass ein Mittelwert dieser Werte verwendet wird. Alternativ ist es ebenfalls denkbar, dass 2, 3, 4, 5, 6, 7, 8, 9, 10, 11, 12, 13, 14, 15, 16, 17, 18, 19, 20 oder mehr Werte unterschiedlicher charakteristischer physikalischer Eigenschaften erfasst werden. Wie viele unterschiedliche charakteristische Eigenschaften des Transformatorenöls zu erfassen sind, unterliegt der fachmännischen Beurteilung. Dies liegt darin begründet, dass eine physikalische Eigenschaft von verschiedenen Faktoren abhängig und/oder beeinflussbar ist, deren zusätzliche Erfassung zu einer weiteren Verbesserung der Bestimmung und/oder Überwachung beiträgt.

In einer Alternative der Weiterbildung der Erfindung ist es denkbar, dass in Schritt c1) mindestens ein Wert der Temperatur des Transformatorenöls erfasst wird.

Die Erfassung der Temperatur ist vorteilhafterweise einfach, schnell und zuverlässig realisierbar. Es ist daher verständlich, dass die Durchschlagspannung des Transformatorenöls basierend auf dem berechneten akustischen Ungleichgewicht und dem mindestens einen erfassten Wert der Temperatur ermittelt wird.

In noch einer Alternative der Weiterbildung der Erfindung ist es denkbar, dass nach dem Schritt c1) ein zusätzlicher Schritt c2) umfasst ist:

c2) Berechnen der Wasserkonzentration und/oder der relativen Sättigung in dem Transformatorenöl.

Im Rahmen der vorliegenden Erfindung ist zudem erkannt worden, dass es zur Fixierung des "Verlagerungspunkts" hilfreich ist, weitere Werte für charakteristische Eigenschaften des Transformatorenöls zu erfassen. Dies liegt darin begründet, dass die Ermittlung der Durchschlagspannung des Transformatorenöls bei einer diagonalen Bewegung des akustischen Ungleichgewichtes, d.h. entlang beider Achsen einer 2-dimensionalen Funktion, d.h. beispielsweise einer Kurve aus Gesamtsäuregehalt und Wasserkonzentration, durch das sich verändernde Transformatorenöl schwierig ist. Daher ist erkannt worden, dass die Ermittlung der Durchschlagspannung durch die direkte Berechnung der Wasserkonzentration und/oder der relativen Sättigung noch einmal drastisch verbessert werden kann. Bevorzugt wird daher in einem weiteren Schritt c2) die Wasserkonzentration und/oder die relative Sättigung in dem Transformatorenöl berechnet.

Weiter bevorzugt ist es diesbezüglich denkbar, zuerst die Löslichkeit von Wasser in Mineralöl zu berechnen. Die Löslichkeit ist definiert als die Gesamtmenge an Wasser, die in dem Mineralöl bei einer bestimmten Temperatur gelöst werden kann. Bevorzugt erfolgt die Berechnung der Löslichkeit von Wasser mittels der Formel (4):

$$\log S_0 = \frac{-1567}{K} + 7{,}0895$$

wobei

$S_0$ die Löslichkeit von Wasser in Mineralöl, und
$K$ die Temperatur in Kelvin (°C + 273) ist.

Weiterhin bevorzugt ist es diesbezüglich denkbar, als nächstes die relative Sättigung des Transformatorenöls zu berechnen. Erfindungsgemäß ist dabei erkannt worden, dass die Berechnung der relativen Sättigung zur schnellen Ermittlung der Durchschlagspannung ausreichend ist. Die relative Sättigung ist definiert als die in dem Transformatorenöl gemessene tatsächliche Wassermenge bezogen auf den Löslichkeitsgrad bei dieser Temperatur. Die in Prozentzahlen ausgedrückte relative Sättigung ist die Wasserkonzentration in dem Öl bezogen auf die Löslichkeit oder die Wasserkonzentration, die das Öl bei der gemessenen Temperatur aufnehmen kann, wie bevorzugt gemäß Formel (5) berechnet:

$$RS = \frac{W_C}{S_0}$$

wobei

$RS$ die relative Sättigung, und
$W_C$ die Wasserkonzentration in ppm, und
$S_0$ die Löslichkeit von Wasser in Mineralöl in ppm ist.

Im Rahmen der Erfindung ist dabei erkannt worden, dass es möglich ist, mithilfe der Formel (5) auf direktem Weg zur Berechnung des Wasserkonzentrationsverhältnisses auf Basis der relativen Sättigung und/oder der bestimmten Temperatur zu gelangen, welche zu einer verbesserten Ermittlung der Durchschlagspannung führt. Bevorzugt erfolgt die Berechnung der Wasserkonzentration mittels der Formel (6):

$$W_C = RS * 10^{\left(\frac{-1567}{K * 7,0895}\right)}$$

wobei

$W_C$ die Wasserkonzentration in ppm, und
$RS$ die relative Sättigung, und
$S_0$ die Löslichkeit von Wasser in Mineralöl in ppm; und
$K$ die Temperatur in Kelvin (°C + 273) ist.

Es ist daher verständlich, dass die Durchschlagspannung des Transformatorenöls basierend auf dem berechneten akustischen Ungleichgewicht, dem erfassten mindestens einen Wert der mindestens einen charakteristischen Eigenschaft, wie der Temperatur, und der berechneten Wasserkonzentration bzw. basierend auf dem berechneten akustischen Ungleichgewicht und der berechneten Wasserkonzentration unter Vernachlässigung der Temperatur ermittelt wird.

In einer weiteren Ausgestaltung ist es denkbar, dass die Impedanz in Schritt a) in 4 Frequenzbändern mit definierter Frequenzbreite von je 75 kHz bestimmt wird. Mittels dieser Ausgestaltung ist es beispielsweise, jedoch keinesfalls ausschließlich möglich, die akustische Spektroskopie in 4 Frequenzbändern mit der definierten Frequenzbreite von je 75 kHz, beispielsweise in dem definierten Frequenzbereich von 75 kHz bis 750 kHz, durchzuführen, beispielsweise in der Art von Frequenzband 1 (125 kHz - 200 kHz), Frequenzband 2 (225 kHz - 300 kHz), Frequenzband 3 (325 kHz - 400 kHz) und Frequenzband 4 (525 kHz - 600 kHz). Bevorzugt wird eine erneute Messung mit der Frequenz durchgeführt, welche der definierten Frequenzbreite von je 75 kHz der Frequenzbänder entspricht,

In einer Weiterbildung der Erfindung ist es denkbar, dass das Verfahren zusätzlich umfasst
e) Darstellen der in Schritt d) durchgeführten Ermittlung.

Mittels dieser Ausgestaltung ist es möglich, die Durchschlagspannung des Transformatorenöls nummerisch und/oder graphisch darzustellen, um auf diese Weise eine Vereinfachung des Verständnisses der Ermittlung in Schritt d) zu erreichen. Einem Fachmann sind geeignete Mittel zur Darstellung einer Ausgabe eines Wertes bekannt. Der Schritt e) kann von einer Ausgabeeinheit unterstützt sein.

Es wird davon ausgegangen, dass die Definitionen und/oder die Ausführungen der oben genannten Begriffe für alle in dieser Beschreibung im Folgenden beschriebenen Aspekte gelten, sofern nichts anderes angegeben ist.

Erfindungsgemäß ist weiterhin eine Vorrichtung zur Bestimmung und/oder Überwachung der Durchschlagspannung eines Transformatorenöls nach einem der Verfahrensansprüche vorgeschlagen, welche Folgendes umfasst:

a) ein erstes Mittel zum Durchführen einer akustischen Impedanzmessung des Transformatorenöls, wobei die Impedanz des ersten, teilweise oder vollständig in dem Transformatorenöl angeordneten, zur Eigen-Schwingung und/oder Schwingungsübertragung auf das Transformatorenöl geeigneten Mittels in mindestens einem Frequenzband definierter Frequenzbreite bestimmbar ist; und
b) mindestens eine Auswerte- und/oder Evaluierungseinheit zum Berechnen eines Resonatorqualitätsfaktors für das Frequenzband, zum Berechnen eines akustischen Ungleichgewichtes des Transformatorenöls und zum Ermitteln der Durchschlagspannung des Transformatorenöls.

Die erfindungsgemäße Vorrichtung ist dabei bevorzugt selbstlernend und/oder selbstkalibrierend, um eine bestmögliche Bestimmung und/oder Überwachung der Durchschlagspannung des Transformatorenöls zu erhalten.

Der Begriff "erstes Mittel" betrifft ein beliebiges, einem Fachmann aus dem Stand der Technik bekanntes Mittel, welches teilweise oder vollständig in dem Transformatorenöl angeordnet und zur Eigen-Schwingung und/oder Schwingungsübertragung auf das Transformatorenöl geeignet ist. Es ist dabei verständlich, dass mindestens eine der Eigenfrequenzen des ersten Mittels im Ultraschallfrequenzbereich liegt, bevorzugt im Bereich des Frequenzbandes. Bevorzugt ist das erste Mittel ein Resonator, ein Resonanzkörper, eine Resonanzkammer oder ein Wandler, wie beispielsweise ein Schallwandler. Weiter bevorzugt ist der Resonator ein oszillierender, piezoelektrischer und/oder aluminiumbeschichteter Resonator. Ein aluminiumbeschichteter piezoelektrischer Resonator bietet den Vorteil, dass dieser für ein Ultraschallrelaxationsverfahren in Bezug auf den Energietransfer zwischen Translations- und Schwingungsfreiheitsgraden zur Messung einer Art von akustischem Ungleichgewicht und direkt für eine Berechnung des Wasser/Gesamtsäure-Gleichgewichts anwendbar ist. Weiterhin bevorzugt ist der Schallwandler ein mechanischer, elektrischer, magnetischer und/oder piezoelektrischer Schallwandler.

Der Begriff "Auswerte- und/oder Evaluierungseinheit" betrifft eine Einheit, welche zum Auswerten, zum Berechnen, zum Vergleichen und/oder zum Ermitteln eines Resonatorqualitätsfaktors für ein Frequenzband, eines akustischen Ungleichgewichtes des Transformatorenöls, mindestens eines Wertes mindestens einer charakteristischen Eigenschaft und/oder der Durchschlagspannung des Transformatorenöls geeignet ist. Geeignete Auswerte- und/oder Evaluierungseinheiten sind einem Fachmann bekannt, beispielsweise ein Computer und/oder ein Computerprogramm. Ein Computerprogramm kann zusätzlich das Ergebnis des Vergleichs beurteilen. Es ist dabei verständlich, dass mehr als nur eine Auswerte- und/oder Evaluierungseinheit umfasst sein kann.

Die erfindungsgemäße Vorrichtung weist den Vorteil auf, dass diese eine hinreichend genaue Sensibilität zur Bestimmung und/oder Überwachung der Durchschlagspannung des Transformatorenöls im laufenden Betrieb, d.h. online, aufweist, sowie zugleich robust genug ist, um unter den alltäglichen Bedingungen eines arbeitenden Transformators langfristig zu bestehen.

Vorteilhafte Weiterbildungen der Erfindung, welche einzeln oder in Kombination realisierbar sind, sind in den Unteransprüchen dargestellt.

In einer Weiterbildung der Erfindung ist es denkbar, dass ein zweites Mittel zum Erfassen mindestens eines Wertes mindestens einer charakteristischen physikalischen Eigenschaft des Transformatorenöls umfasst ist.

Der Begriff "zweites Mittel" betrifft ein beliebiges, einem Fachmann aus dem Stand der Technik bekanntes Mittel, welches dazu geeignet ist, mindestens einen Wert mindestens einer charakteristischen physikalischen Eigenschaft des Transformatorenöls zu mindestens einem Zeitpunkt zu erfassen, wie beispielsweise die Dichte, die Farbe, der Brechungsindex, die Temperatur, die Löslichkeit in Wasser, die Wasserkonzentration, die Oberflächenspannung, die Viskosität, die relative und/oder absolute Feuchte bzw. Sättigung, den Verlustfaktor, den Säuregehalt, die elektrische Konstante, die elektrische Leitfähigkeit und/oder die Konzentration mindestens eines Fluides. Zudem sind weitere, hier nicht aufgeführte, Eigenschaften denkbar. Bevorzugt erfolgt gleichzeitig die Erfassung des Zeitpunktes. Bevorzugt ist das zweite Mittel ein Sensor, wie ein Druck-, Feuchte- und/oder Temperatursensor.

In noch einer Weiterbildung der Erfindung ist es denkbar, dass eine Ausgabeeinheit zur Darstellung der mittels der Auswerte- und/oder Evaluierungseinheit durchgeführten Ermittlung umfasst ist.

Der Begriff "Ausgabeeinheit" betrifft eine Einheit, welche zur Darstellung der ausgewerteten, berechneten, verglichenen und/oder ermittelten Werte, Ergebnisse und/oder der Durchschlagspannung des Transformatorenöls geeignet ist. Mittels dieser Ausgestaltung ist es möglich, die Werte, die Ergebnisse und/oder die Durchschlagspannung des Transformatorenöls nummerisch und/oder graphisch darzustellen, um auf diese Weise eine Vereinfachung des Verständnisses der Ermittlung im Schritt b) zu erreichen. Einem Fachmann ist eine geeignete Ausgabeeinheit zur Darstellung bekannt.

In einer weiteren Weiterbildung der vorliegenden Erfindung ist es denkbar, dass das erste Mittel, das zweite Mittel, die Auswerte- und/oder Evaluierungseinheit und/oder die Ausgabeeinheit in einem Bauteil anordenbar sind. Diese Ausgestaltung bietet den Vorteil, dass die Vorrichtung kompakt und sehr leicht händelbar sowie gut transportierbar ist.

In einer alternativen Ausgestaltung dieser Weiterbildung ist es denkbar, dass das Bauteil eine Messkammer, ein Stick und/oder ein Adapter ist. Diese Ausgestaltung bietet den Vorteil, dass die Vorrichtung einfach, schnell und zuverlässig mit einer Vorrichtung in der Hochspannungstechnik, wie beispielsweise einem Transformator, verbindbar ist. Weiterhin bevorzugt ist die Verbindung der Vorrichtung direkt, beispielsweise kabelgebunden oder über einen Adapter.

In einer weiteren Ausgestaltung ist es denkbar, dass die Vorrichtung eine Heizeinrichtung umfasst. Diese Ausgestaltung bietet den Vorteil, dass die Vorrichtung vor Durchführung der ersten Messung aufheizbar ist, so dass sichergestellt ist, dass die in der Vorrichtung befindlichen Mittel immer trocken sind. Dies trägt maßgeblich zur Verbesserung der gemessenen Werte bei, da Verfälschungen in der Regel aufgrund einer Durchfeuchtung der Mittel bedingt sind. Einem Fachmann sind derartige Heizeinrichtungen, wie beispielsweise eine Heizschlange und/oder ein Peltier-Element, gut bekannt.

Erfindungsgemäß ist weiterhin eine Vorrichtung in der Hochspannungstechnik, insbesondere ein Transformator, ein Kondensator, eine Petersenspule und/oder ein Schalter, umfassend Transformatorenöl und eine Einrichtung zur Verbindung mit der Vorrichtung nach einem der vorhergehenden Ansprüche vorgeschlagen, wobei die Verbindung direkt erfolgt.

Der Begriff "direkte Verbindung" betrifft eine beliebige, unmittelbare Verbindung der Einrichtung mit der Vorrichtung. Eine derartige Verbindung ist beispielsweise, jedoch keineswegs ausschließlich, mittels einer Ausnehmung und/oder eines Vorsprungs seitens der Einrichtung und einer entsprechend ausgebildeten Vorrichtung realisierbar. Es ist weiterhin denkbar, dass eine direkte Verbindung eine USB-, TCP/IP-, MODBUS-Verbindung oder eine andere kabelgebundene oder kabellose Verbindung ist. Mittels dieser Ausgestaltung ist es einfach, schnell und zuverlässig möglich, die Vorrichtung zur Bestimmung der Durchschlagspannung des Transformatorenöls mit einer Vorrichtung in der Hochspannungstechnik zu verbinden, um schnell, zuverlässig und hinreichend genau den Zustand derselben zu bestimmen und/oder zu überwachen.

Weitere Einzelheiten, Merkmale und Vorteile der Erfindung ergeben sich aus der nachfolgenden Beschreibung der bevorzugten Ausführungsbeispiele in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigen:

Fig. 1 eine isometrische Darstellung einer ersten Ausführungsform einer erfindungsgemäßen Vorrichtung mit Schutzabdeckung; und

Fig. 2 eine weitere isometrische Darstellung der ersten Ausführungsform einer erfindungsgemäßen Vorrichtung ohne Schutzabdeckung; und

Fig. 3 eine Explosionsdarstellung der ersten Ausführungsform einer erfindungsgemäßen Vorrichtung ohne Schutzabdeckung; und

Fig. 4 eine isometrische Darstellung des Sensorbereichs der ersten Ausführungsform einer erfindungsgemäßen Vorrichtung mit unterschiedlichen Befestigungsbereichen (Fig. 4a und 4b); und

Fig. 5 eine isometrische Darstellung einer zweiten Ausführungsform einer erfindungsgemäßen Vorrichtung; und

Fig. 6 eine isometrische Darstellung eines Sensorbereichs der zweiten Ausführungsform einer erfindungsgemäßen Vorrichtung; und

Fig. 7 eine weitere isometrische Darstellung eines Sensorbereichs der zweiten Ausführungsform einer erfindungsgemäßen Vorrichtung; und

Fig. 8 eine Matrix-Grafik einer beispielhaften Berechnung der Bestimmung der Durchschlagspannung eines Transformatorenöls; und

Fig. 9 einen Regressionsbaum für die Berechnung der Durchschlagspannung bei mehreren untersuchten Transformatorenölen; und

Fig. 10 eine 2-dimensionale Funktion aus dem Gesamtsäuregehalt (TAN) und der Wasserkonzentration ($W_C$) mehrerer untersuchter Transformatorenölproben; und

Fig. 11 eine Spektraldichtefunktion eines aluminiumbeschichteten piezoelektrischen Resonators in Kontakt mit einem Transformatorenöl; und

Fig. 12 eine Polynom-Approximation für die Durchschlagspannung (Werte zur Berechnung exponentiell skaliert) auf Basis bestimmter Wasserkonzentration-Werte und akustischer Ungleichgewichts-Werte.

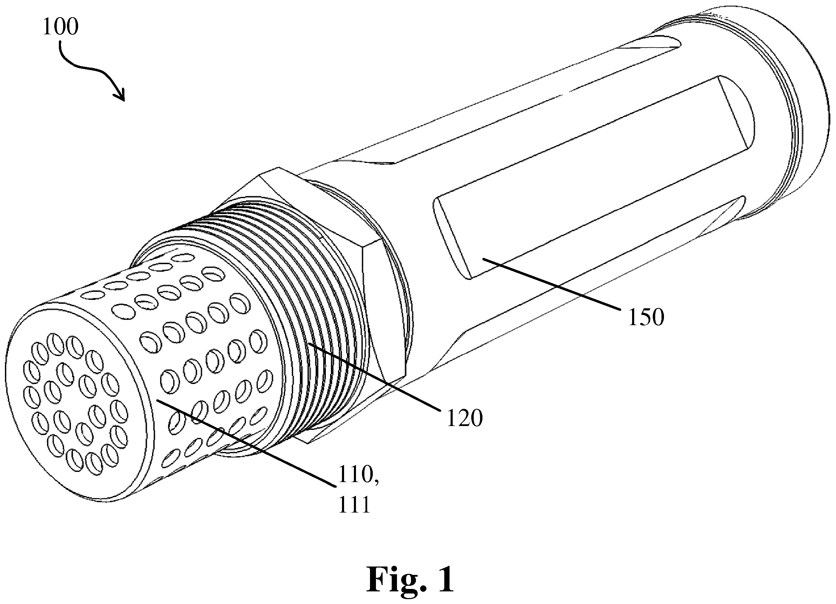

In der **Fig. 1** ist eine erste Ausführungsform einer erfindungsgemäßen Vorrichtung 100 zur Bestimmung und/oder Überwachung der Durchschlagspannung eines Transformatorenöls in isometrischer Darstellung gezeigt, welche in der Form eines Sticks 100 ausgebildet ist. Wie deutlich in der Fig. 1 zu erkennen ist, weist der Stick 100 einen mit einer Schutzabdeckung 111 versehenen Sensorbereich 110 auf. Zudem ist an dem Stick 100 ein Befestigungsbereich 120 zur sicheren Befestigung des Sticks 100 an einem Transformator vorgesehen, welcher beispielsweise in der Form eines 1,5-Zoll-Rohrgewindes ausgebildet ist. Das Gehäuse 150 des Sticks 100 dient neben der Isolierung auch dem Schutz der Elektronik, insbesondere vor einem ungewollten elektrischen und/oder elektromagnetischen Effekt, sowie als Kommunikationsvorrichtung und ist aus einem beliebigen Material denkbar. Bevorzugt ist das Gehäuse 105 aus einem die elektromagnetische Verträglichkeit gewährleistenden metallischen Werkstoff.

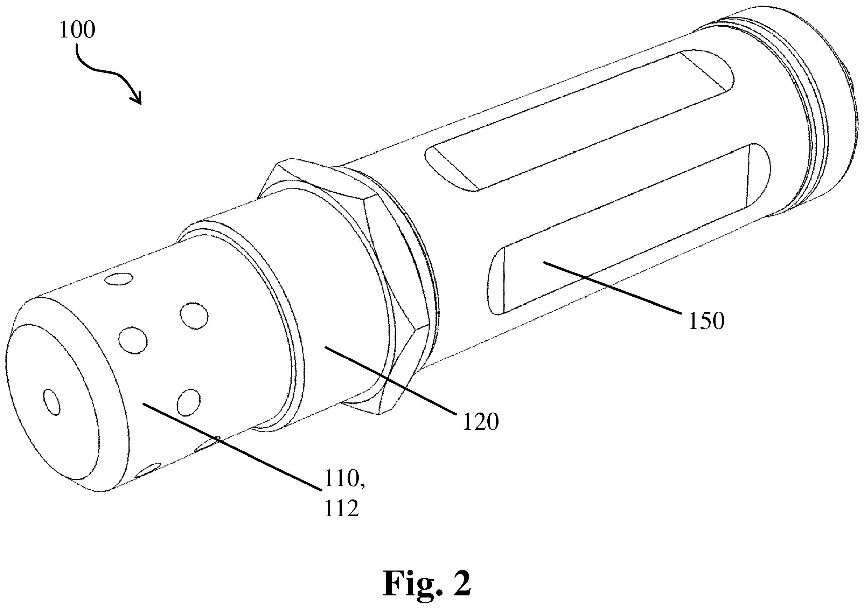

In der **Fig. 2** ist eine alternative Ausgestaltung der ersten Ausführungsform einer erfindungsgemäßen Vorrichtung 100 zur Bestimmung und/oder Überwachung der Durchschlagspannung eines Transformatorenöls gezeigt, welche ebenfalls in der Form eines Sticks 100 ausgebildet ist. Wie deutlich in der Fig. 2 zu erkennen ist, weist der Stick 100 einen Sensorbereich 110 mit einer Resonanzkammer 112 auf. Diese ist zu erkennen, da in der alternativen Ausgestaltung der ersten Ausführungsform keine Schutzabdeckung gezeigt ist. Der Stick 100 weist zudem einen Befestigungsbereich 120 zur sicheren Befestigung des Sticks 100 an einem Transformator auf, welcher in dieser Alternative beispielsweise als 1,0-Zoll-Rohrgewinde ausgebildet ist. Ebenfalls ist ein Gehäuse 150 umfasst.

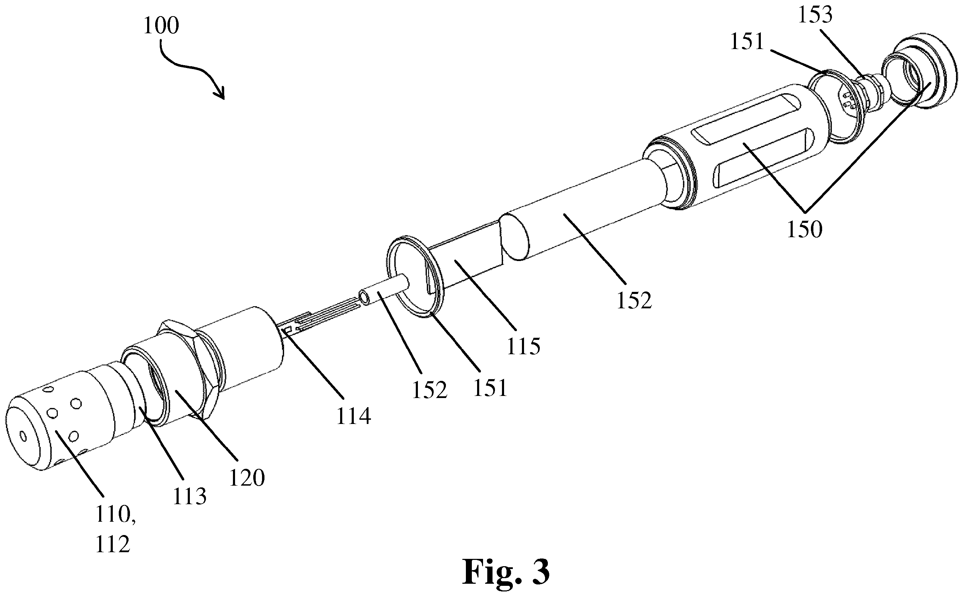

In der **Fig. 3** ist eine Explosionsdarstellung der alternativen Ausgestaltung der ersten Ausführungsform der erfindungsgemäßen Vorrichtung 100 nach Fig. 2 gezeigt, welche die innere Struktur der Vorrichtung 100 zeigt. Wie deutlich zu erkennen ist, sind kritische und empfindliche Komponenten der Vorrichtung 100 durch ein robustes und ausgeklügeltes Zusammenspiel von Anordnung und Gehäuseelementen 150, 151 und 152 abgedeckt und geschützt. Dieser Stick 100 weist ebenfalls einen Sensorbereich 110 auf sowie eine aufgrund der nicht dargestellten Schutzabdeckung sichtbare Resonanzkammer 112. Des Weiteren ist ein akustischer Wandler 113 und ein Befestigungsbereich 120 umfasst, welcher, wie ebenfalls in Fig. 2 gezeigt, beispielsweise in der Form eines 1,0-Zoll-Rohrgewindes ausgebildet ist.

Des Weiteren ist in Fig. 3 deutlich zu erkennen, dass ein Feuchte- und/oder Temperatur-Sensor 114 sowie der Resonator in der Resonanzkammer 112 und dem akustischen Wandler 113 aufgenommen sind. Zudem ist die entsprechende Elektronik 115 umfasst. Die Elektronik 115 ist von einer Isolierung 152, beispielsweise aus einem Kunststoff, umgeben. Das Gehäuse 150 umfasst zudem mehrere Isolierelemente 152, welche beispielsweise aus Kunststoff herstellbar sind. Des Weiteren sind mehrere Distanzringe 151 und eine Kabelbuchse 153 umfasst, um, beispielsweise über Modbus, eine Verbindung mit der Sensorelektronik 115 des Stick 100 zu ermöglichen.

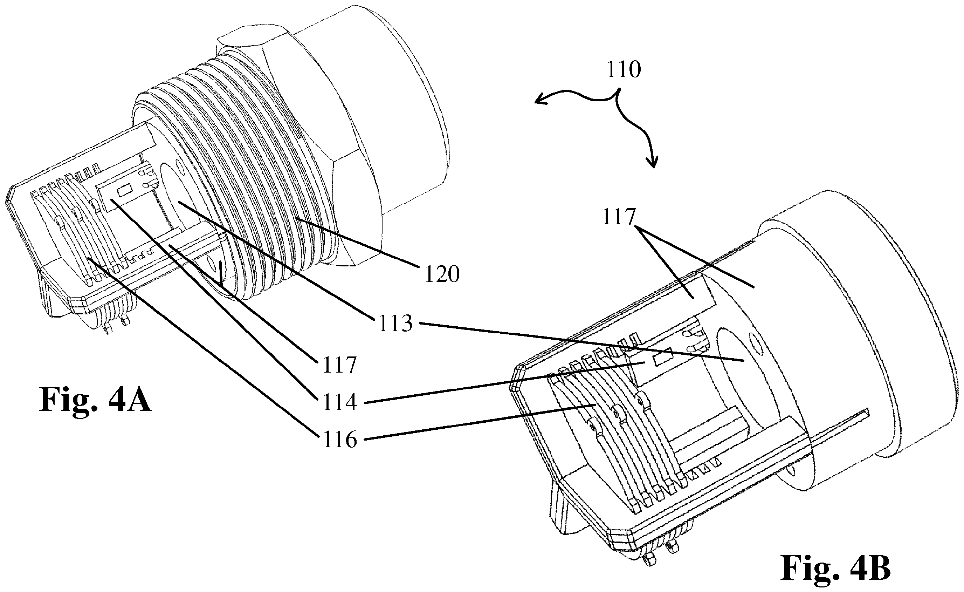

In der **Fig. 4** sind zwei alternative Ausgestaltungen einer ersten Ausführungsform des Sensorbereichs 110 der erfindungsgemäßen Vorrichtung 100 gezeigt. Wie deutlich in **Fig. 4a** und **Fig. 4b** zu erkennen ist, umfasst der Sensorbereich 110 mehrere Kondensatorplatten 116, welche Teil eines dielektrischen Sensors sind. Des Weiteren ist ein Feuchte- und/oder Temperatur-Sensor 114 und ein akustischer Wandler mit Resonanzkammer 113 umfasst. Das Halteelement 117 ist beispielsweise isolierend und aus einem Kunststoff herstellbar. In der Fig. 4a ist zudem ein Befestigungsbereich 120 dargestellt, welcher beispielsweise in der Form eines 1,5-Zoll-Rohrgewindes, ebenso wie in Fig. 1, ausgebildet ist.

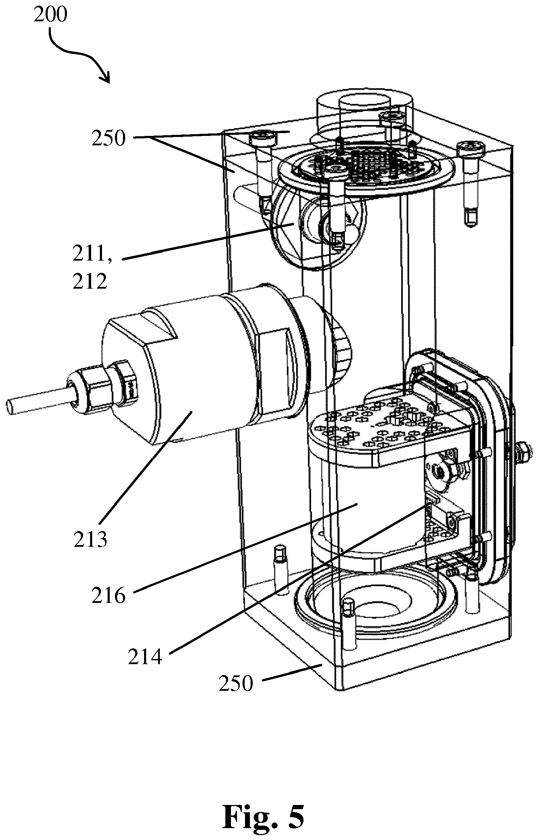

In der **Fig. 5** ist eine zweite Ausführungsform einer erfindungsgemäßen Vorrichtung 200 zur Bestimmung und/oder Überwachung der Durchschlagspannung eines Transformatorenöls gezeigt, welche in der Form einer Messkammer 200 ausgebildet ist. Wie deutlich in Fig. 5 zu erkennen ist, weist die Messkammer 200 mehrere Sensorbereiche auf, so ist beispielsweise in einem Sensorbereich ein Dichte- und/oder Viskositäts-Sensor 211 und/oder ein optischer Sensor 212 angeordnet. In einem weiteren Sensorbereich ist ein akustischer Sensor 213 angeordnet sowie weiterhin ein dielektrischer Sensor 216 und ein Feuchte- und/oder Temperatur-Sensor 214. Die Messkammer 200 weist zudem ein Gehäuse 250 auf.

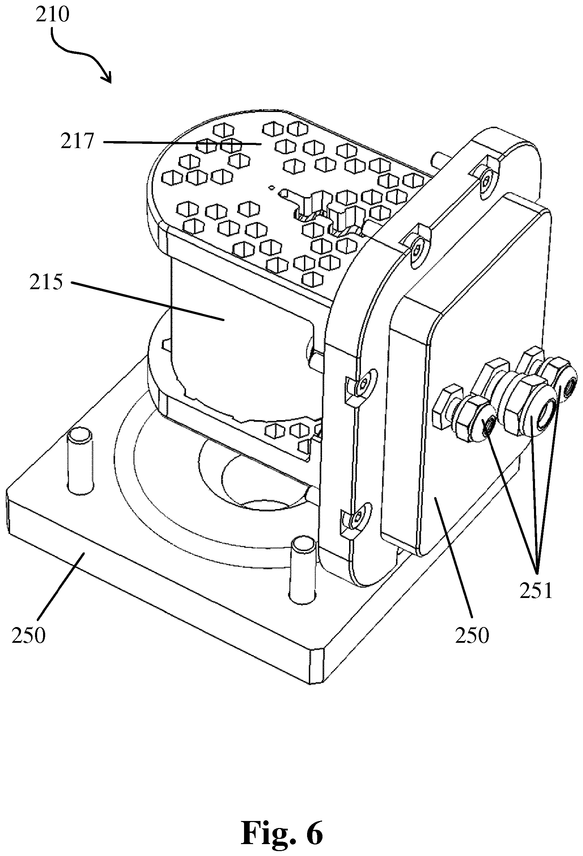

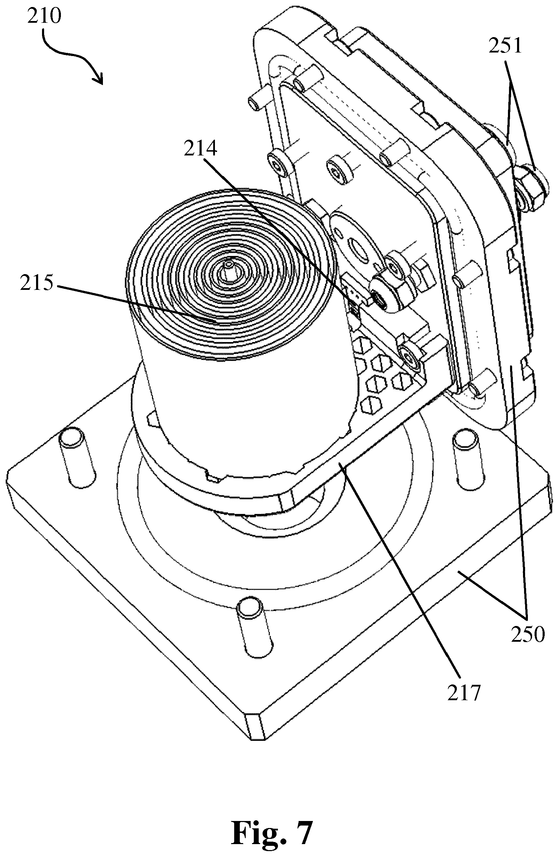

In den **Fig. 6** und **Fig. 7** ist einer der Sensorbereiche 210 der Messkammer nach Fig. 5 im Detail dargestellt. Wie deutlich in den Fig. 6 und 7 dargestellt, ist ein Halteelement 217 umfasst, welches isolierend ist und beispielsweise aus einem Kunststoff herstellbar ist. Zudem sind in beiden Figuren deutlich die Kondensatorplatten 215 (ausgeführt als Zylinderkondensator aus konzentrischen Rohren) zu erkennen, welche Teil des dielektrischen Sensors sind. In der Fig. 7 ist zudem der Feuchte- und/oder Temperatur-Sensor 214 zu erkennen. Weiterhin ist in den Fig. 6 und Fig. 7 das der Isolierung der Messkammer aus Fig. 5 dienende Gehäuse 250 nebst den Kabeldurchführungen 251 zur Verbindung mit dieser sichtbar.

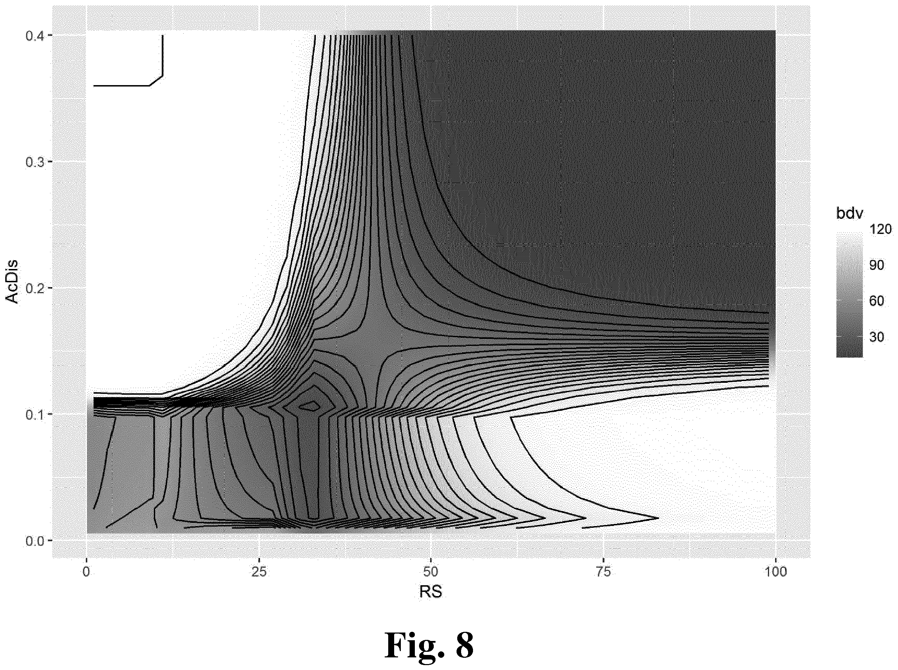

In der **Fig. 8** ist eine graphische Darstellung in Form einer Matrix-Grafik für ein beispielhaftes Berechnungsbeispiel der Bestimmung der Durchschlagspannung (BDV; eng. *break down voltage)* eines Transformatorenöls basierend auf einer 2-dimensionalen Funktion aus relativer Feuchte (RS; eng. *relative saturation)* und akustischem Ungleichgewicht (AcDis; eng. *acustic disbalance)* gezeigt. Die in der Matrix dargestellten Isogene entsprechen je 5 [kV].

In der ersten Stufe der 2-dimensionalen Funktion wird die Funktion *bdvL (RS, AcDis)* berechnet, welche von der Hauptfunktion h(x) abhängig ist. Diesbezüglich gilt die folgende Formel (1):

$$h(x) = \begin{cases} x & wenn\ x \geq 0 \\ 0 & wenn\ x < 0 \end{cases}$$

wobei

$h$ die Hauptfunktion, und

$x$ der Argumentenwert ist.

Gemäß Formel (1) hat die Hauptfunktion unterschiedliche Argumente. Wenn der Argumentenwert $x \geq 0$ ist, dann übernimmt die Funktion diesen Wert. Ist der Argumentenwert $x < 0$, dann ist der Wert null und dieses Gleichungsglied wird gelöscht.

Ausgehend von dieser Formel (1) ist nachfolgend in Formel (2) ein beispielhaftes Berechnungsbeispiel der Bestimmung der Durchschlagspannung (BDV) gezeigt. Die Formel (2) lautet:

$$
\begin{aligned}
bdvL\ (RS,\ AcDis) = {}& -0.10 - \\
& 0.23h(0.096885 - \log_{10}(RS)) - \\
& 96.79\ h(\log_{10}(RS) - 0.986885) - \\
& 9.38\ h(\log_{10}(RS) - 1.03756) - \\
& 19.27\ h(\log_{10}(RS) - 1.43403) + \\
& 30.27\ h(\log_{10}(RS) - 1.51121) + \\
& 0.21\ h(-0.987312 - \log_{10}(AcDis)) + \\
& 67.11\ h(\log_{10}(AcDis) + 0.987312) - \\
& 169.59\ h(\log_{10}(RS) - 0.986885)*\ h(\log_{10}(AcDis) + 1.44532) + \\
& 169.36\ h(\log_{10}(RS) - 0.986885 * h(-1.44532 - \log_{10}(AcDis)) - \\
& 119.70\ h(\log_{10}(RS) - 0.986885) * h(\log_{10}(AcDis) + 0.996463) + \\
& 179.58\ h(\log_{10}(RS) - 0.986885 * h(\log_{10}(AcDis) + 1.99022) + \\
& 0.02\ h(1.04391 - \log_{10}(RS)) * h(-0.987312 - \log_{10}(AcDis)) + \\
& 13.10\ h(\log_{10}(RS) - 1.04391) * h(-0.987312 - \log_{10}(AcDis)) + \\
& 11.55\ h(\log10(RS) - 1.43403) * h(\log10(AcDis) + 2.00147
\end{aligned}
$$

wobei

$bdvL$ ein nicht normierter Zwischenwert der Durchschlagspannung,
$h$ die Hauptfunktion mit den Argumentenwerten x,
wobei

$x = RS$ die relative Feuchte, und
$x = AcDis$ die akustische Disbalance ist.

In der zweiten Stufe der 2-dimensionalen Funktion wird der mittels Formel (2) berechnete Wert *bdvL* gemäß der geltenden Norm DIN EN 60243-1:2012-05 normiert (siehe "Elektrische Durchschlagfestigkeit von isolierenden Werkstoffen - Prüfverfahren - Teil 1: Prüfungen bei technischen Frequenzen" (IEC 112/199/CDV:2011)). Die Normierung erfolgt gemäß der Formel (3):

$$BDV = 10 + \frac{110}{1 + \exp(-bdvL)} \; [kV]$$

wobei

$bdvL$ ein nicht normierter Zwischenwert der Durchschlagspannung, und $BDV$ die Durchschlagspannung ist.

Weitere Informationen sind dem bekannten Standardwerk Friedman (1991) Multivariate Adaptive Regression Splines (with discussion) Annals of Statistics 19/1, 1-141, zu entnehmen (https://statistics.stanford.edu/research/multivariate-adaptive-regressionsplines).

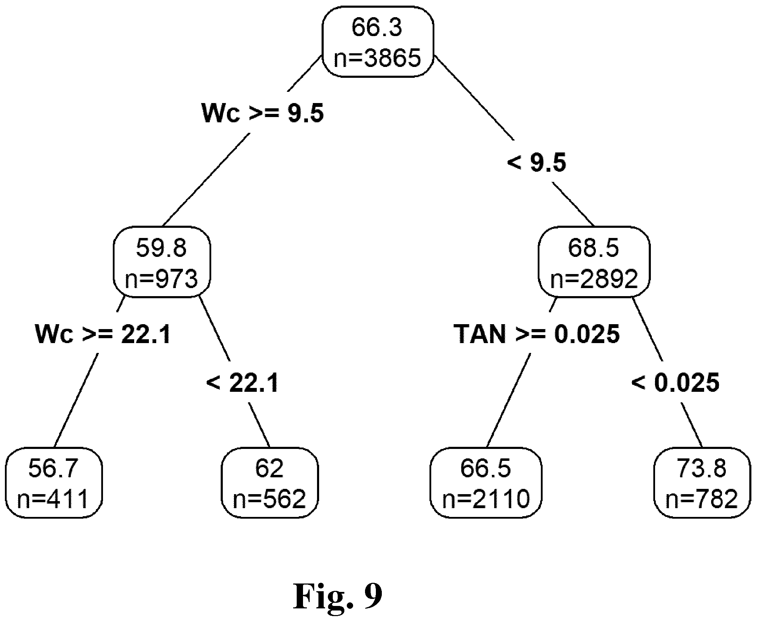

In der **Fig. 9** ist ein Überblick über das Verfahren und die Vorrichtung gezeigt. Mittels dieses Überblicks kann gezeigt werden, dass es zu einer drastisch verbesserten Ermittlung der Durchschlagspannung (BDV, engl.: *breakdown voltage)* ausreicht, lediglich zwei der beschriebenen Parameter zu messen, da zwischen Wasserkonzentration ($W_C$, engl. *water content),* Gesamtsäurezahl (TAN, engl. *total-acid number)* und Durchschlagspannung bei den untersuchten Tranformatorölpproben eine enge Korrelation besteht. Unter Auswertung von über 3800 Laborproben aus mehr als 900 Transformatoren kann der in Fig. 9 dargestellte Regressionsbaum für die Durchschlagspannung aufgestellt werden, welcher die berechneten BDV-Werte mit einer Fehlerquote von unter 10 % beim BDV-Wert zeigt. Damit ist die erfindungsgemäße Ermittlung sogar den Ergebnissen aus dem mittels standardmäßiger BDV-Laborausrüstung durchgeführten Verfahren nach IEC 60243-1 überlegen, welches eine Fehlerquote von bis zu 20 % aufweist. D.h., dass mittels des erfindungsgemäßen Verfahrens und der Vorrichtung die Durchschlagspannung sehr gut berechenbar und somit hinreichend genau bestimmbar ist. Dabei ist in Fig. 9 die obere Zahl im Feld, z. B. 66,3, der BDV-Wert in [kV], die untere Zahl ist die entsprechende Anzahl der Proben, n = 3865. Die Regeln, z. B. $W_C$ >= 9,5 [ppm], zeigen die Entscheidungsbedingungen für den Schritt auf die nächste Stufe in der Entscheidungshierarchie. Einheiten in der Fig. 9 sind BDV [kV], $W_C$ [ppm], TAN [mg/kg; KOH].

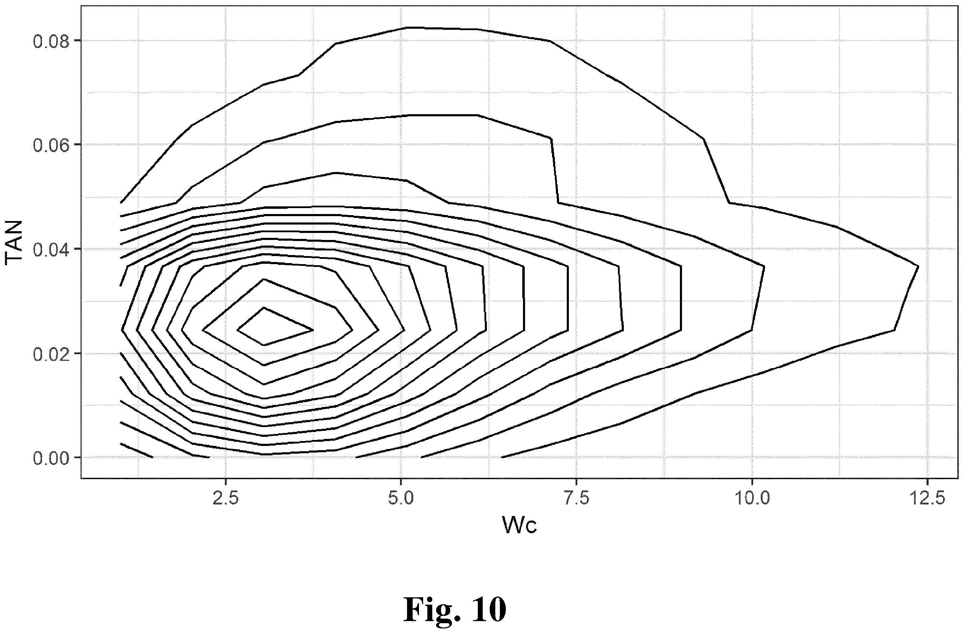

**Fig. 10** zeigt eine 2-dimensionale Funktion aus dem Gesamtsäuregehalt (TAN in mg/kg; KOH) und der Wasserkonzentration ($W_C$ in ppm) mehrerer untersuchter Transformatorenölproben. Dabei ist in Fig. 10 insbesondere der interessanteste $W_C$/TAN-Bereich, in welchem sich nahezu 75 % aller untersuchter Proben sammelten, gezeigt.

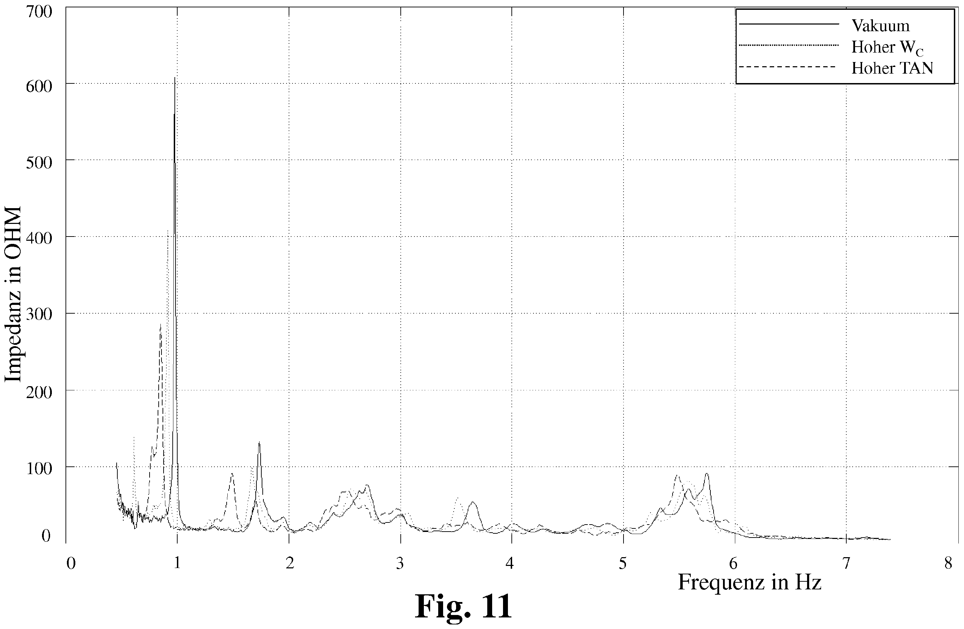

In **Fig. 11** ist die Spektraldichtefunktion dargestellt, welche das unterschiedliche Verhalten eines aluminiumbeschichteten piezoelektrischen Resonators in Kontakt mit dem Transformatorenöl zeigt. Der Oszillationsbereich liegt zwischen 75 kHz und 750 kHz. Dieser Impedanzkurve sind dabei fünf eindeutige Bereiche zu entnehmen, welche bei der Berechnung des akustischen Ungleichgewichtes (AcDis) berücksichtigt werden.

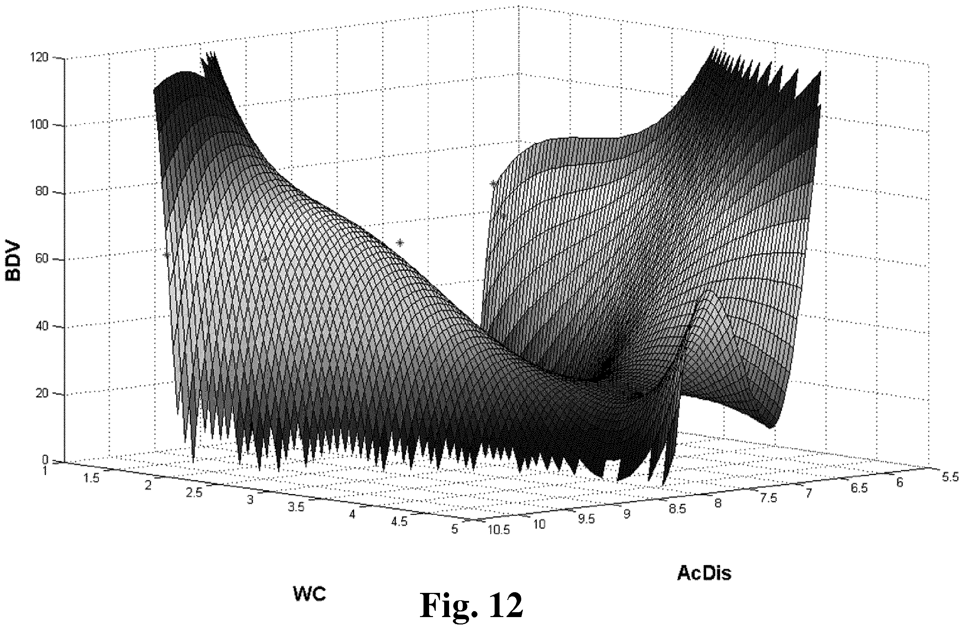

In **Fig. 12** ist eine Polynom-Approximation für BDV auf Grundlage der gemessenen Labor- und Sensordaten zu Durchschlagspannung (BDV), Wasserkonzentration ($W_C$), Temperatur (TEMP), relative Sättigung (RS), Gesamtsäurezahl (TAN), Oberflächenspannung (IFT) und akustischem Ungleichgewicht (AcDis) aller Laborproben gezeigt, wobei die BDV-Daten zur Berechnung zunächst normiert und als Exponenten in eine angepasste MATLAB™-Regressionsfunktion übernommen wurden, um auf Basis bestimmter $W_C$- und AcDis-Werte zu einem empirischen ermittelten Modell der BDV zu gelangen. Der Restfehler in Fig. 12 liegt im Bereich von 2,5 %.

Erfindungsgemäß wurden die $W_C$-Daten wurden dabei gemäß der Formel (6) aus gemessenen TEMP- und RS-Daten errechnet. Daraus ergab sich die folgende Matrix (7) für die BDV-Berechnung:

$$BDV \, (W_C, AcDis) \; = \; ln \, (W^T * Q * A)$$

wobei

$W = [1, W_C, W_C^2, W_C^3]^T$, und
$A = [1, AcDis, AcDis^2, AcDis^3]^T$

oder

$$BDV \, (Wc, AcDis) = ln \left( \sum_{0 \le p,q \le 3} Q_{p,q} \cdot Wc^p \cdot AcDis^q \right)$$

und

$$Q = \begin{bmatrix} -774{,}719 & 13668{,}212 & -62493{,}001 & 91578{,}430 \\ 3463{,}366 & -58357{,}859 & 263056{,}913 & -382222{,}994 \\ -5099{,}993 & 82366{,}071 & -366206{,}210 & 528174{,}797 \\ 2461{,}998 & -38292{,}458 & 168221{,}373 & -241289{,}917 \end{bmatrix}$$

ist.

Es ist offensichtlich, dass derartige Zahlen ohne weiteres von einem 32-Bit-Embedded-System mit Gleitkommaprozessor (FPU) verarbeitet werden können. Zur Vermeidung von Problemen mit schweren und sehr zeitintensiven Matrixberechnungen, für die ein Embedded-Prozessor nur begrenzt geeignet ist, sowie mit der Grenzinstabilität des vorgestellten Modells, wurde für diese Lösung eine Lookup-Tabelle aufgestellt. Es hat sich dabei gezeigt, dass eine Lookup-Tabelle eine sehr adäquate Darstellung der Matrixlösung ist und zudem das Grenzverhalten der Transformatorölproben umfasst. Daher erweist sich eine Lookup-Tabelle als erste Wahl hinsichtlich Geschwindigkeit, Auflösung und Stabilität. Zusammenfassend bleibt festzuhalten, dass die Berechnung der Durchschlagspannung (BDV) einen Gesamtrestfehler von unter 3,71 % aufweist, was ein hervorragender Wert ist.

**Bezugszeichenliste**

| | |
|---|---|
| 100 | Stick |
| 110 | Sensorbereich |
| 111 | Schutzabdeckung |
| 112 | Resonanzkammer |
| 113 | Akustischer Wandler |
| 114 | Feuchte- und/oder Temperatur-Sensor |
| 115 | Elektronik |
| 116 | Kondensatorplatten |
| 117 | Halteelement |
| 120 | Befestigungsbereich |
| 150 | Gehäuse |
| 151 | Distanzring |
| 152 | Isolierelement |
| 153 | Kabelbuchse |

| | |
|---|---|
| 200 | Messkammer |
| 210 | Sensorbereich |
| 211 | Dichte- und/oder Viskositäts-Sensor |
| 212 | Optischer Sensor |
| 213 | Akustischer Sensor |
| 214 | Feuchte- und/oder Temperatur-Sensor |
| 215 | Kondensatorplatten |
| 216 | Dielektrischer Sensor |
| 217 | Halteelement |
| 250 | Gehäuse |
| 251 | Kabeldurchführung |

**Patentansprüche**

1.  Verfahren zur Bestimmung und/oder Überwachung der Durchschlagspannung eines Transformatorenöls, umfassend,

    a) Durchführen einer akustischen Impedanzmessung des Transformatorenöls, wobei die Impedanz eines teilweise oder vollständig in dem Transformatorenöl angeordneten, zur Eigen-Schwingung und/oder Schwingungsübertragung auf das Transformatorenöl geeigneten Mittels in mindestens einem Frequenzband definierter Frequenzbreite bestimmt wird; und
    b) Berechnen eines Resonatorqualitätsfaktors für das Frequenzband basierend auf der in Schritt a) durchgeführten Bestimmung; und

c) Durchführen einer Kalibrierung und Berechnen eines akustischen Ungleichgewichtes des Transformatorenöls basierend auf den in der Kalibrierung ermittelten Kalibrierwerten und auf der in Schritt b) durchgeführten Berechnung; und

d) Ermitteln der Durchschlagspannung des Transformatorenöls basierend auf der in Schritt c) durchgeführten Berechnung.

**2.** Verfahren nach Anspruch 1,
wobei nach dem Schritt c) ein zusätzlicher Schritt c1) umfasst ist:
c1) Erfassen mindestens eines Wertes mindestens einer charakteristischen physikalischen Eigenschaft des Transformatorenöls.

**3.** Verfahren nach Anspruch 2,
wobei in Schritt c1) mindestens ein Wert der Temperatur des Transformatorenöls erfasst wird.

**4.** Verfahren nach Anspruch 2 oder 3,
wobei nach dem Schritt c1) ein zusätzlicher Schritt c2) umfasst ist:
c2) Berechnen der Wasserkonzentration und/oder der relativen Sättigung in dem Transformatorenöl.

**5.** Verfahren nach einem der Ansprüche 1 bis 4,
wobei die Impedanz in Schritt a) in 4 Frequenzbändern mit definierter Frequenzbreite von je 75 kHz bestimmt wird.

**6.** Verfahren nach einem der Ansprüche 1 bis 5,
wobei das Verfahren zusätzlich umfasst:
e) Darstellen der in Schritt d) durchgeführten Ermittlung.

**7.** Vorrichtung (100, 200) zur Bestimmung und/oder Überwachung der Durchschlagspannung eines Transformatorenöls nach einem der vorhergehenden Verfahrensansprüche, umfassend

a) ein erstes Mittel (110, 112, 113, 210, 211, 212, 213) zum Durchführen einer akustischen Impedanzmessung des Transformatorenöls, wobei die Impedanz des ersten, teilweise oder vollständig in dem Transformatorenöl angeordneten, zur Eigen-Schwingung und/oder Schwingungsübertragung auf das Transformatorenöl geeigneten Mittels (110, 112, 113, 210, 211, 212, 213) in mindestens einem Frequenzband definierter Frequenzbreite bestimmbar ist; und

b) mindestens eine Auswerte- und/oder Evaluierungseinheit zum Berechnen eines Resonatorqualitätsfaktors für das Frequenzband, zum Durchführen einer Kalibrierung und zum Berechnen eines akustischen Ungleichgewichtes des Transformatorenöls basierend auf den in der Kalibrierung ermittelten Kalibrierwerten und zum Ermitteln der Durchschlagspannung des Transformatorenöls.

**8.** Vorrichtung (100, 200) nach Anspruch 7,
wobei ein zweites Mittel (110, 114, 116, 210, 214, 215, 216) zum Erfassen mindestens eines Wertes mindestens einer charakteristischen physikalischen Eigenschaft des Transformatorenöls umfasst ist.

**9.** Vorrichtung (100, 200) nach Anspruch 7 oder 8,
wobei eine Ausgabeeinheit zur Darstellung der mittels der Auswerte- und/oder Evaluierungseinheit durchgeführten Ermittlung umfasst ist.

**10.** Vorrichtung (100, 200) nach einem der Ansprüche 7 bis 9,
wobei das erste Mittel (110, 112, 113, 210, 211, 212, 213), das zweite Mittel (110, 114, 116, 210, 214, 215, 216), die Auswerte- und/oder Evaluierungseinheit und/oder die Ausgabeeinheit in einem Bauteil anordenbar sind.

**11.** Vorrichtung (100, 200) nach Anspruch 10, wobei das Bauteil eine Messkammer (200), ein Stick (100) und/oder ein Adapter ist.

**12.** Vorrichtung (100, 200) nach einem der Ansprüche 7 bis 11,
wobei die Vorrichtung (100, 200) eine Heizeinrichtung umfasst.

**13.** Vorrichtung in der Hochspannungstechnik umfassend Transformatorenöl, eine Vorrichtung (100, 200) nach einem der Ansprüche 7 bis 12 und eine Einrichtung zur Verbindung mit der Vorrichtung (100, 200), wobei die Verbindung direkt erfolgt.

14. Vorrichtung in der Hochspannungstechnik nach Anspruch 13, wobei die Vorrichtung in der Hochspannungstechnik ein Transformator, ein Kondensator, eine Petersenspule und/oder ein Schalter ist.

**Claims**

1. A method for determining and/or monitoring the breakdown voltage of a transformer oil, comprising the steps of

   a) performing an acoustic impedance measurement of the transformer oil, the impedance of a medium partially or entirely disposed in the transformer oil and capable of naturally vibrating and/or transmitting vibrations to the transformer oil being determined in at least one frequency band of defined frequency width; and
   b) calculating a resonator quality factor for the frequency band based on the determination performed in step a); and
   c) performing a calibration process and calculating an acoustic disbalance of the transformer oil based on the calibration values ascertained in the calibration process and on the calculation performed in step b); and
   d) ascertaining the breakdown voltage of the transformer oil based on the calculation performed in step c).

2. The method according to claim 1,
   wherein the method comprises an additional step c1) after step c):
   c1) registering at least one value of at least one characteristic physical property of the transformer oil.

3. The method according to claim 2,
   wherein at least one value of the temperature of the transformer oil is registered in step c1).

4. The method according to claim 2 or 3,
   wherein the method comprises an additional step c2) after step c1):
   c2) calculating the water content and/or the relative saturation in the transformer oil.

5. The method according to any one of claims 1 to 4,
   wherein, in step a), the impedance is determined in four frequency bands each having a defined frequency width of 75 kHz.

6. The method according to any one of claims 1 to 5,
   wherein the method additionally comprises the step of: e) displaying the ascertainment performed in step d).

7. A device (100, 200) for determining and/or monitoring the breakdown voltage of a transformer oil according to any one of the preceding method claims, comprising

   a) a first medium (110, 112, 113, 210, 211, 212, 213) for performing an acoustic impedance measurement of the transformer oil, the impedance of the first medium (110, 112, 113, 210, 211, 212, 213), which is partially or entirely disposed in the transformer oil and capable of naturally vibrating and/or transmitting vibrations to the transformer oil, is determined in at least one frequency band of defined frequency width; and
   b) at least one analyzing and/or evaluating unit for calculating a resonator quality factor for the frequency band, for performing a calibration process and for calculating an acoustic disbalance of the transformer oil based on the calibration values ascertained in the calibration process and for ascertaining the breakdown voltage of the transformer oil.

8. The device (100, 200) according to claim 7,
   wherein the device (100, 200) comprises a second medium (110, 114, 116, 210, 214, 215, 216) for registering at least one value of at least one characteristic physical property of the transformer oil.

9. The device (100, 200) according to claim 7 or 8,
   wherein the device (100, 200) comprises an output unit for displaying the ascertainment performed by means of the analyzing and/or evaluating unit.

**10.** The device (100, 200) according to any one of claims 7 to 9,
wherein the first medium (110, 112, 113, 210, 211, 212, 213), the second medium (110, 114, 116, 210, 214, 215, 216), the analyzing and/or evaluating unit and/or the output unit are/is disposed in one component.

**11.** The device (100, 200) according to claim 10, wherein the component is a measuring chamber (200), a stick (100) and/or an adapter.

**12.** The device (100, 200) according to any one of claims 7 to 11,
wherein the device (100, 200) comprises a heating device.

**13.** A device of high-voltage technology, comprising transformer oil, a device (100, 200) according to any one of claims 7 to 12 and a means for connection to the device (100, 200), the connection being a direct connection.

**14.** The device of high-voltage technology according to claim 13, the device of high-voltage technology being a transformer, a capacitor, a Petersen coil and/or a switch.

**Revendications**

**1.** Procédé pour déterminer et/ou contrôler la tension de claquage d'une huile de transformateur, comprenant les étapes suivantes :

a) effectuer une mesure de l'impédance acoustique de l'huile de transformateur, l'impédance d'un milieu disposé partiellement ou totalement dans l'huile de transformateur et capable de vibrer naturellement et/ou de transmettre des vibrations à l'huile de transformateur étant déterminée dans au moins une bande de fréquences de largueur de fréquence définie ; et
b) calculer un facteur de qualité du résonateur pour la bande de fréquences sur la base de la détermination effectuée dans l'étape a) ; et
c) effectuer un processus d'étalonnage et calculer un déséquilibre acoustique de l'huile de transformateur sur la base des valeurs d'étalonnage déterminées dans le processus d'étalonnage et sur la base du calcul effectué dans l'étape b) ; et
d) déterminer la tension de claquage de l'huile de transformateur sur la base du calcul effectué dans l'étape c).

**2.** Procédé selon la revendication 1,
dans lequel le procédé comprend une étape supplémentaire c1) après l'étape c) :
c1) saisir au moins une valeur d'au moins une propriété physique caractéristique de l'huile de transformateur.

**3.** Procédé selon la revendication 2,
dans lequel au moins une valeur de la température de l'huile de transformateur est saisie dans l'étape c1).

**4.** Procédé selon la revendication 2 ou 3,
dans lequel le procédé comprend une étape supplémentaire c2) après l'étape c1) :
c2) calculer la teneur en eau et/ou la saturation relative dans l'huile de transformateur.

**5.** Procédé selon l'une quelconque des revendications 1 à 4,
dans lequel, dans l'étape a), l'impédance est déterminée dans quatre bandes de fréquences dont chacune a une largeur de fréquence définie de 75 kHz.

**6.** Procédé selon l'une quelconque des revendications 1 à 5,
dans lequel le procédé comprend supplémentairement l'étape consistant à :
e) afficher la détermination effectuée dans l'étape d).

**7.** Dispositif (100, 200) pour déterminer et/ou contrôler la tension de claquage d'une huile de transformateur selon l'une quelconque des revendications de procédé précédentes, comprenant :

a) un premier milieu (110, 112, 113, 210, 211, 212, 213) pour effectuer une mesure de l'impédance acoustique de l'huile de transformateur, l'impédance du premier milieu (110, 112, 113, 210, 211, 212, 213) disposé partiellement ou totalement dans l'huile de transformateur et capable de vibrer naturellement et/ou de transmettre des vibrations à l'huile de transformateur étant déterminée dans au moins une bande de fréquences de largeur de fréquence définie ; et

b) au moins une unité d'analyse et/ou d'évaluation pour calculer un facteur de qualité du résonateur pour la bande de fréquences, pour effectuer un processus d'étalonnage et pour calculer un déséquilibre acoustique de l'huile de transformateur sur la base des valeurs d'étalonnage déterminées dans le processus d'étalonnage et pour déterminer la tension de claquage de l'huile de transformateur.

8.  Dispositif (100, 200) selon la revendication 7,
    dans lequel le dispositif (100, 200) comprend un deuxième milieu (110, 114, 116, 210, 214, 215, 216) pour saisir au moins une valeur d'au moins une propriété physique caractéristique de l'huile de transformateur.

9.  Dispositif (100, 200) selon la revendication 7 ou 8,
    dans lequel le dispositif (100, 200) comprend une unité de sortie pour afficher la détermination effectuée au moyen de l'unité d'analyse et/ou d'évaluation.

10. Dispositif (100, 200) selon l'une quelconque des revendications 7 à 9,
    dans lequel le premier milieu (110, 112, 113, 210, 211, 212, 213), le deuxième milieu (110, 114, 116, 210, 214, 215, 216), l'unité d'analyse et/ou d'évaluation et/ou l'unité de sortie sont/est disposé(s) dans un composant.

11. Dispositif (100, 200) selon la revendication 10, dans lequel le composant est une chambre de mesure (200), un bâton (100) et/ou un adaptateur.

12. Dispositif (100, 200) selon l'une quelconque des revendications 7 à 11, dans lequel le dispositif (100, 200) comprend un dispositif de chauffage.

13. Dispositif de la technique de la haute tension, comprenant de l'huile de transformateur, un dispositif (100, 200) selon l'une quelconque des revendications 7 à 12 et un moyen pour la connexion au dispositif (100, 200), la connexion étant une connexion directe.

14. Dispositif de la technique de la haute tension selon la revendication 13, dans lequel le dispositif de la technique de la haute tension est un transformateur, un condensateur, une bobine Petersen et/ou un commutateur.

Fig. 1

EP 3 513 154 B1

**Fig. 2**

EP 3 513 154 B1

**Fig. 3**

**Fig. 4A**

**Fig. 4B**

110

120

117

113

117

114

116

EP 3 513 154 B1

**Fig. 5**

23

**Fig. 6**

**Fig. 7**

Fig. 8

**Fig. 9**

EP 3 513 154 B1

EP 3 513 154 B1

**Fig. 10**

**Fig. 11**

EP 3 513 154 B1

EP 3 513 154 B1

**Fig. 12**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19706486 A1
- DE 102014104963 A1

- DE 102013005003 A1